(19) Europäisches Patentamt
European Patent Office
Office européen des brevets

(11) Publication number: **0 074 543 A1**

# (12) EUROPEAN PATENT APPLICATION

(21) Application number: 82107892.0

(22) Date of filing: 27.08.82

(51) Int. Cl.³: C 07 C 35/31
C 11 B 9/00, A 61 K 7/46
C 07 C 29/40

(30) Priority: 15.09.81 CH 5942/81

(43) Date of publication of application:
23.03.83 Bulletin 83/12

(84) Designated Contracting States:
CH DE FR GB LI NL

(71) Applicant: Roure Bertrand Dupont Société Anonyme
55, Voie des Bans
F-95102 Argenteuil(FR)

(72) Inventor: Maupetit, Pierre
"Le Mas St. Claude" Avenue Sidi Brahim
F-06130 Grasse(FR)

(74) Representative: Lederer, Franz, Dr. et al,
Patentanwälte Dr. Lederer Franz Meyer-Roxlau Reiner F.
Lucile-Grahn-Strasse 22
D-8000 München 80(DE)

## (54) Bicyclic alcohols, a process for their preparation and odoriferous compositions containing them.

(57) The invention relates to bicyclic alcohols of the general formula

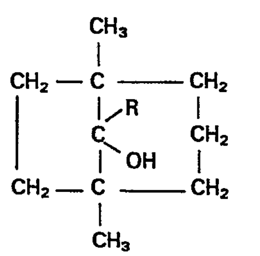

I

wherein R represents a hydrocarbon group having from 1 to 7 carbon atoms. The compounds are useful as odoriferous agents in that they have a Patchouli type odour. A process for the preparation of the compounds is also disclosed.

EP 0 074 543 A1

Société Anonyme Roure Bertrand Dupont
55, Voie des Bans, F-95102 Argenteuil, France

0074548

Ref. 6600/32

# Bicyclic alcohols, a process for their preparation and odoriferous compositions containing them

This invention relates to bicyclic alcohols, a process for their preparation and to odoriferous compositions containing them.

The compounds according to the invention are represented by the general formula

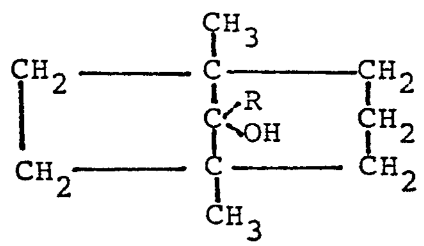

I

wherein R represents a hydrocarbon group having from 1 to 7 carbon atoms.

The hydrocarbon group preferably contains from 1 to 6 carbon atoms. The hydrocarbon group may be saturated or unsaturated, straight chain or branched, for example alkyl, alkenyl or alkynyl as well as aryl or aralkyl such as phenyl or benzyl. Specific examples of compounds of the general formula I include those wherein R represents methyl, ethyl, propyl, sec.butyl, n-butyl, isobutyl, tert.-butyl, pentyl, isopentyl or hexyl; allyl, vinyl, propenyl, ethynyl, phenyl or benzyl.

The compounds according to the invention may be prepared by any convenient process. One such process is characterised in that one reacts the ketone of the formula

Ke/ 3.8.82

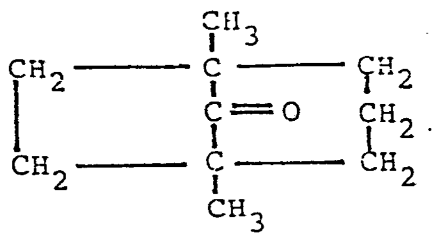

II

with an organo-metallic compound which donates the radical R. The organo-metallic compound may be a Grignard compound of the formula

R-Mg-X

wherein X represents a halogen atom preferably chlorine, bromine or iodine or an alkaline organometallic compound such as the acetylide of sodium, potassium or lithium, or vinyl lithium.

The compounds of formula I where the radical R is a saturated aliphatic group may in addition be prepared by hydrogenation of a compound of formula I wherein the radical R is not saturated. This hydrogenation may be carried out by conventional methods.

The starting material ketone of formula II is a known substance and may be prepared by conventional methods.

The compounds of formula I possess exceptional odoriferous properties and are characterised by having an odour approximating closely to the original woody odour of Patchouli oil. Since this natural product is used to a considerable extent in the perfume industry, the compounds of the present invention may consequently be used for the preparation of perfumes and perfumed products, such as soaps, liquid and solid detergents, perfumed industrial products, aerosols, cosmetic products of all kinds, for example toilet water, pommades, beauty creams, face creams, lipsticks, salts and bath oils.

The powerful odour and tenacity of the compounds of the present invention considered within the general make up of Patchouli oil, present wide possibilities as regards the preparation of new perfume formulations. These compounds confer to mixtures to which they are added, considerable odoriferous powers and also accentuate the odours of other compounds e.g. the head notes for hesperides and agrest notes and bottom notes for woody, amber and musc aromas such as occur in vetyver, oak moss, benjoin and muscs.

Specific compounds according to the present invention have the following odours:

| Compound | Odour |
|---|---|
| 1,5,8-Trimethyl-bicyclo-[3.2.1]-octan-8-ol | Woody, agreste, very powerful characteristic of Patchouli oil |
| 8-Ethynyl-1,5-dimethyl-bicyclo[3.2.1]-octan-8-ol | Earthy and camphor like, dry and woody allied to the odour of Patchouli oil |
| 8-Allyl-1,5-dimethyl-bicyclo[3.2.1]-octan-8-ol | Woody having Patchouli oil notes |

The amount of odoriferous product according to the invention which may be employed can vary over a wide range depending on the product which is to be perfumed and on the type and amount of other constituents present in the odoriferous composition. The amount used may for example be from 1% to 3% in toilet waters and cosmetic products and from 5 to 10% in soaps detergents and alcohol based perfumes.

The compounds of formula I can exist in different stereoisomeric forms and in addition in two different conformational structures. Formula I covers all these possible variations.

The invention will now be illustrated with reference to the following Examples.

Example 1

6 g of magnesium turnings were introduced into a 500 ml reactor equipped with a stirrer, heating jacket, dropping funnel and condensor. Over 1 hour a solution of 35.5 g of methyl iodide in 175 ml of diethyl ether was added under reflux. The reaction mass was then cooled to room temperature and a solution of 30.4 g of 1,5-dimethyl-bicyclo[3.2.1]-octan-8-one in 75 ml of anhydrous diethyl ether was added. Following the addition, the mixture was refluxed for two hours. The product was then cooled and poured into 300 ml of aqueous ammonium chloride (10% w/v). After extraction of the organic phase with diethyl ether, washing to neutrality with water and distillation there was obtained 32.0 g of crude product which after redistillation yielded 29.9 g of crystalline 1,5,8-trimethylbicyclo-[3.2.1]-octan-8-ol having a melting point of 35°C.

Example 2

101 g of 85% potassium carbonate in powder form in 560 ml of toluene were introduced into a reactor. 504 ml of dimethyl sulfoxide were then added. The mixture was heated to 80°C for 30 minutes and then cooled to 0°C. Acetylene was then led in and 30 g of 1,5-dimethylbicyclo-[3.2.1]-octan-8-one were added over 30 minutes. The mixture was then shaken for 16 hours during which time the acetylene introduction was continued. The reaction mass was then poured onto a cold aqueous salt solution (10% w/v). After extraction of the organic phase with toluene and distillation there was obtained 232.8 g of a crude product which on redistillation yielded 206.8 g of crystalline 8-ethynyl-1,5-dimethylbicyclo[3.2.1]-octan-8-ol having a melting point of 25°C.

Example 3

136.8 g of 8-ethynyl-1,5-dimethylbicyclo[3.2.1]-octan-8-ol, 525 ml of ethanol and 2.5 g of palladium on carbon (5% w/w) were placed in a 1 litre autoclave. Hydrogenation was then effected at a presure of 20 bar at the ambient temperature for 2 hours 30 minutes. The catalyst was then filtered off and the alcohol distilled off. There was obtained 140 g of 8-ethyl-1,5-dimethylbicyclo[3.2.1]-octan-8-ol having a boiling point of 64°C (0.8 mm Hg).

Example 4

15.0 g of magnesium turnings and then a few ml of a solution of 72.6 g of allyl bromide in 440 ml of diethyl ether were placed in a 2 liter vessel. After initiation of the reaction the mass was cooled to around 5°C and the remainder of the allyl bromide solution was added over 4.5 hours. The mass was cooled to around -15°C and a solution of 38.0 g of 1,5-dimethylbicyclo[3.2.1]-octan-8-one in 40 ml of diethyl ether was added over 1 hour. The mass was then stirred for 30 minutes and poured into 500 ml of aqueous ammonium chloride (10% w/v). After extraction of the organic phase with diethyl ether, washing to neutrality with water and distillation off of the solvent there was obtained 45.0 g of crude product which on fractional distillation yielded 45.0 g of 8-allyl-1,5-dimethylbicyclo[3.2.1]-octan-8-ol having a boiling point of 78°C (1 mm Hg).

## Example 5

Odoriferous composition

| | |
|---|---|
| Amyl salicylate | 80 |
| Cedarwood oil | 50 |
| Acetylated cedarwood terpenes | 100 |
| Clove oil | 20 |
| Coumarin | 20 |
| Geranium oil | 10 |
| Lavander oil | 290 |
| γ-methyl ionone | 60 |
| Reconstituted moss | 10 |
| Java vetiver oil | 40 |
| Patchouli oil | 80 |
| Terpeneless bergamot oil | 200 |
| Linalyl acetate | 20 |
| 8-Ethyl-1,5-dimethylbicyclo-[3.2.1]-octan-8-ol | 20 |
| | 1000 |

## Example 6

| | |
|---|---|
| Terpenless lemon grass oil | 100 |
| Tyrolean oak moss absolute | 60 |
| Coumarin | 10 |
| 12-Oxa-16-hexadecanolide | 60 |
| Cinnamic alcohol | 40 |
| Linalool | 16 |
| Linalyl acetate | 80 |
| Terpeneless bergamot oil | 160 |
| γ-methyl ionone | 24 |
| Eugenol | 20 |
| Ylang-Ylang oil | 20 |
| Jasmin absolute | 20 |
| Benjoin resin No. 1 | 80 |
| 1,5,5-trimethyl-4-(1-formyloxy-ethyl)-cyclohex-1-ene | 40 |

| | |
|---|---|
| Vetyveryl acetate | 80 |
| Dipropylene glycol | 100 |
| 1,5,8-Trimethylbicyclo[3.2.1]-octan-8-ol | 90 |
| | 1000 |

CLAIMS

1. Compounds of the general formula

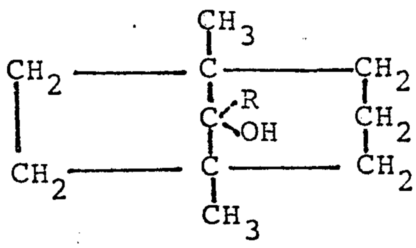

I

wherein R represents a hydrocarbon group having from 1 to 7 carbon atoms.

2. Compounds according to claim 1, characterised in that R represents a hydrocarbon group having from 1 to 6 carbon atoms.

3. Compounds according to claim 1 or claim 2, characterised in that R represents an alkyl, alkenyl, alkynyl or aryl group.

4. Compounds according to any one of claims 1 to 3, characterised in that R represents a methyl, ethyl, propyl, sec.butyl, n-butyl, isobutyl, t-butyl, pentyl, isopentyl, hexyl, allyl, vinyl, propenyl, ethynyl or benzyl group.

5. 1,5,8-Trimethylbicyclo[3.2.1]-octan-8-ol; 8-ethynyl-1,5-dimethylbicyclo[3.2.1]-octan-8-ol; 8-ethyl-1,5-dimethylbicyclo[3.2.1]-octan-8-ol or 8-allyl-1,5-dimethylbicyclo-[3.2.1]-octan-8-ol.

6. Process for the preparation of compounds of the general formula

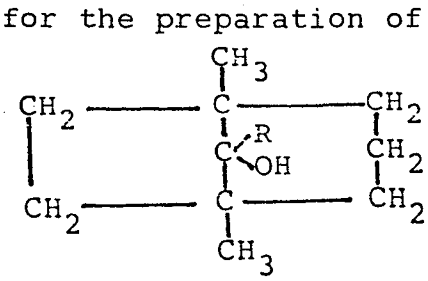

I

wherein R represents a hydrocarbon group containing from 1 to 7 carbon atoms characterised in that a ketone of the formula

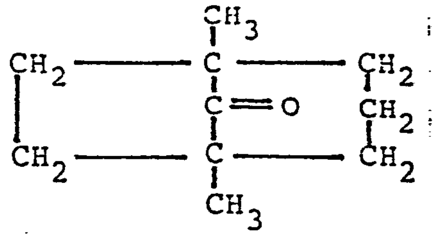

II

is reacted with an organo-metallic compound which donates the radical R.

7. A process according to claim 6, characterised in that the organo-metallic compound is a Grignard reagent having the formula

R-Mg-X

wherein R represents a hydrocarbon group having from 1 to 7 carbon atoms and X represents a halogen atom.

8. A process for the preparation of compounds of the general formula

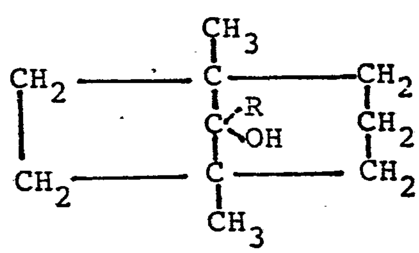

I

wherein R represents a saturated aliphatic hydrocarbon group having from 2 to 7 carbon atoms, characterised in that a compound of the formula I wherein R represents an unsaturated hydrocarbon group having from 2 to 7 carbon atoms is hydrogenated.

9. A process for perfuming compositions or for the modification of the odour of odoriferous compositions, characterised in that a compound of the general formula

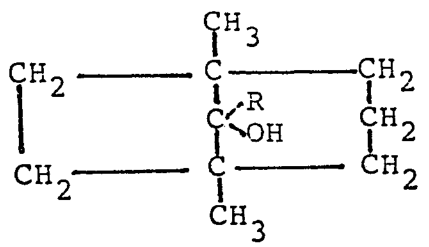

I

wherein R represents a hydrocarbon group having from 1 to 7 carbon atoms,

is incorporated into said composition.

10. An odoriferous composition characterised in that it contains a compound of the general formula

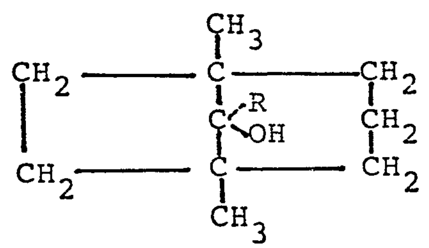

I

wherein R represents a hydrocarbon group having from 1 to 7 carbon atoms.

11. The use of compounds of the general formula

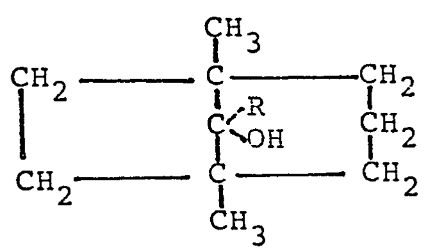

I

wherein R represents a hydrocarbon group having from 1 to 7 carbon atoms

as an odoriferous agent.

***

European Patent Office

# EUROPEAN SEARCH REPORT

0074543
Application number

EP 82 10 7892

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | GB-A-2 020 659 (SHELL)<br>* Claims 1,20 *<br>--- | 1,9-11 | C 07 C 35/31<br>C 11 B 9/00<br>A 61 K 7/46<br>C 07 C 29/40 |
| A | FR-A-2 441 604 (NAARDEN & SHELL AROMA)<br>* Claims 1,15 *<br>----- | 1,9-11 | |
| | | | TECHNICAL FIELDS SEARCHED (Int. Cl. 3) |
| | | | C 07 C 35/00<br>C 11 B 9/00 |

The present search report has been drawn up for all claims

| Place of search | Date of completion of the search | Examiner |
|---|---|---|
| THE HAGUE | 18-10-1982 | DELHOMME H.A. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03 82